# EUROPEAN PATENT APPLICATION

(11) **EP 2 420 249 A1**
(43) Date of publication of application: **22.02.2012**
(21) Application number: 11183836.3
(22) Date of filing: 05.08.2004
(51) Int. Cl.: A61K 39/39, A61K 38/19, A61K 39/00

(54) **Vaccine immunotherapy for immune suppressed patients**

(30) Priority: 08.08.2003 US 637869
(62) Divisional of application: 04780361.4
(71) Applicant: IRX Therapeutics, Inc., New York, NY 10019 (US)
(72) Inventor: Hadden, John, Cold Springs Harbor, NY 11724 (US)
(74) Representative: Austin, Hedley William

(57) **Abstract**

Use of a natural cytokine mixture (NCM) including the cytokines IL-1, IL-2, IL-6, IL-8, IL-12, IFN-gamma, TNF-alpha, GM-CSF and G-CSF, for immunotherapy of a cancer patient in a dosage regime comprising
(i) administration of the NCM in an inactive amount, and
(ii) administration of Indomethacin and cyclophosphamide.

## Description

The present invention, which is divided from EP04780361.4 (granted as No 1653912) relates to vaccine therapy for cancer patients

It has become increasingly apparent that human cancers have antigens, which if reacted upon by the host's immune systems, lead to tumour regression. These antigens have been defined by both serological and cellular immune approaches. This has lead to the definition of both B and T cell epitopes (Sahin U, et al, Curr Opin Immunol 9:709-715, 1997; Van der Eynde, B, et al. Curr Opin Immunol 9:684-693, 1997; Wang RF, et al., Immunologic Reviews 170:85-100, 1999). Based upon these results, it has become a goal of cancer immunotherapists to induce regressions of tumours. However, historically, successful efforts have been sporadic and generally minor in frequency and magnitude.

A fundamental problem in the effort to immunise cancer patients is that the tumour-bearing state is associated with immunosuppressive mechanisms derived from both the tumour and from the host's disturbed immune system (Kavanaugh D Y, et al, Hematol-Oncol Clinics of North Amer 10(4):927-951, 1996), thereby making immunisation difficult and until now impossible on a consistent basis. Immune suppression or depletion involves a reduced capacity of the immune system to respond. Such suppression can be drug or disease induced. The condition can be drug induced by treatment, virus induced as in AIDS, or induced by a disease state such as cancer. The immune system in this condition is effectively turned off.

A variety of tumour immunisation strategies have been developed. However, all such strategies are complex and deviate significantly from the conventional immunisation strategies used for infectious diseases (Weber J. Tumor, Medscape Anthology 3:2, 2000). One such tumour immunization strategy involves a material sold under the trade mark Theratope, a Sialyl TN polysaccharide mucin antigen conjugated with keyhole limpet hemocyanine and administered with Detox mycobacterium adjuvant (Detox is a trade mark) and low dose cyclophosphamide (Maclean G D, et al, J Immunother Emphasis Tumor Immunol 19(4):309-316, 1996). However, use of this vaccine in patients with metastatic breast and ovarian cancer has yielded major clinical responses in a low percentage of patients. A major response means greater than 50% tumour reduction.

Gene therapy has also been attempted using an adenovirus construct as an expression vector for genes expressing Papilloma virus peptide 16 has been used for immunization or patients with cervical cancer and has yielded major clinical responses in a low percentage of patients (Borysiewickz L K, et al, Lancet 347:1524-1527, 1996).

Dendritic cell mediated therapy has also been attempted, in which dendritic cells were pulsed with oligopeptide fragments of prostate specific antigens (PSA). Prostate specific membrane antigen (PSMA) has been used in patients with metastatic prostate cancer with major clinical responses in a low percentage of patients (Sanda M G, et al, Urology 52:2, 1999; Murphy GP, et al, The Prostate. 38:43-78, 1999).

Additionally, autologous tumours have been used with low dose cyclophosphamide and BCG to immunize cancer patients with malignant melanoma. However, few clinical responses were reported (Mastrangelo M J, et al, Seminars in Oncology 23(6):773-781, 1996). Another strategy attempted included using MAGE antigens with a variety of vaccine adjuvants. Again, this has yielded few, if any, responses in patients with malignant melanoma.

US Patents 5503841; 5800810; 6060068; 5643565 and 5100664 (all in the name of Doyle) disclose methods of enhancing the immune response in patients using Interleukin 2-(IL-2). This method is disclosed for use in response to infectious diseases and primarily functions using antigens known to be immunogenic

As disclosed above, the treatment of cancer is known to require different approaches. To date, treatment with IL-2 has shown minor effects in two cancers, renal cell and malignant melanoma (response rates less than 20%). It is generally considered ineffective in head and neck squamous cell cancer (H&NSCC); in cervical cancer and in prostate cancer. Hence, it is not approved for these uses. It would therefore not be obvious based on the method of the above patents to use of small peptides in the treatment of cancer.

It is important to contrast prevention with known "classic" antigens of complex structure and high molecular weights in healthy patients vs. treatment (generally unsuccessful) with tumour antigens or peptides (general unsuccessful) in immunosupressed patients (generally unsuccessful). The first is easy and our current viral vaccines attest to their efficacy. The latter is nearly impossible on a routine basis despite 30 years of intense effort.

Immunisation has now been proposed with endogenous peptide processed and presented by dendritic cells or endogenously administered to an environment (lymph node) where dendritic cells have been prepared and can present them to T-cells effectively. This was once considered to be an insurmountable challenge. Peptides are much too small to be effective immunogens; their half life is short they are often non-mutated self antigens to which the patient is immunologically tolerant and gaining a response is tantamount to inducing auto immunity.

In several of the above strategies, cellular and/or tumoral immunity to tumour-associated antigens has been induced (Weber J. Tumor, Medscape Anthology 3:2, 2000; Maclean G D, et al, J Immunother Emphasis Tumor Immunol 19(4):309-316,1996; Borysiewickz L K, et al, Lancet 347:1524-1527, 1996; Sanda M G, et al, Urology 52:2,1999). This is especially so in association with tumour regression. Nevertheless, the success rate of such treatments is negligible and inconsistent (<30%).

NCM, a non-recombinant cytokine mixture, is now known to be active in unblocking immunisation at a regional lymph node. NCM, as defined in US Patents 5632983 and 5698194, is (in brief) prepared in the continuous presence of a 4-aminoquinolone antibiotic and with the continuous or pulsed presence of a mitogen, which in the preferred embodiment is PHA.

WO02/34119 and WO03/035004 both disclose the use of such a natural cytokine mixture (NCM), which includes the cytokines IL-1, IL-2, IL-6, IL-8, IL-12, IFN-gamma, TNF alpha, G-CSF and GM-CSF, for the treatment of cancer patients, generally by injection, resulting in maturation of immature dendritic cells in regional lymph nodes. These documents give a great deal of information concerning how the NCM may be administered (typically by injection around lymphatics that drain into lymph nodes), in some embodiments together with thymosin α1. The NCM may further be administered together with cyclophosphamide (CY) and a non-steroidal antiinflammatory drug (NSAID) such as indomethacin (INDO).

Detailed instructions and examples are given in both documents of modes of administration and modes of action to immunosuppressed patients, for example, together with an "adjuvant" which can enhance the immune response to a particular antigen, and together with a n exogenous or endogenous tumour associated antigen.

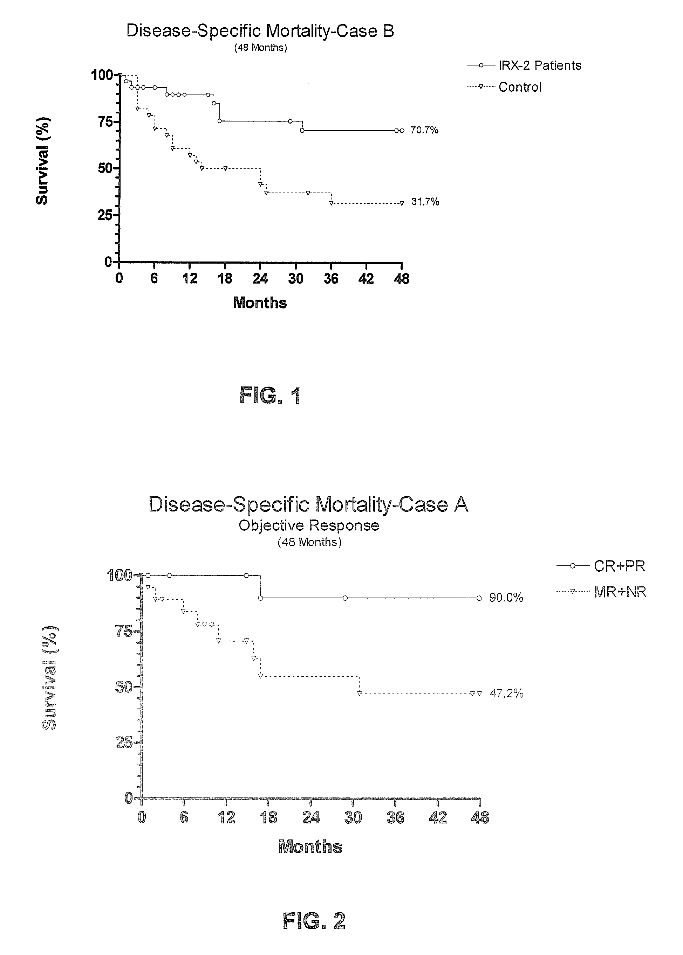

In the accompanying drawings, Figure 1, which is a graph illustrating the survival percentage of treated patients at 48 months, shows that treatment of patients with NCM (referred to as IRX-2) is associated with increased survival at 48 months (p < 0.01).

Figure 2 is a graph illustrating the survival of complete and partial responders compared to minor and non-responders, as will be explained herein. The figure shows that clinical responses determine survival, in that patients with complete response (CR) and partial response (PR) (>50% tumour reduction) have better survival rate than those with minor responses (MR) (<50%, but >24% tumour reduction) or no response (NR) (<25%)(p<0.01).

Figure 3, which is a graph illustrating the relationship of pathology index to survival, shows that patients with stronger pathological responses (index of 6 to 9) have better survival rate than those with weaker pathological responses (<6) (p<0.02).

Figure 4, which is a graph showing the relationship of lymphocyte infiltration to survival shows that lymphoid infiltration into the tumour as a single variable predicts survival.

Figure 5 is a graph illustrating the survival percentage (dose response) of treated patients at 24 months, wherein "x" is equal to about 100 IU/mL of IL-2.

Chi Square analysis of the relationship of clinical response to the pathological response in the above figures shows a highly significant relationship (p<0.01) indicating that the two are coordinately related to each other as well as to survival and thus provide a statistical triangulation of the data interrelating clinical responses, immune regression parameters, and survival. Such relationships have never been shown for immunotherapy of a human cancer.

This has led to the surprising finding that it can be advantageous to administer the NCM in an inactive amount, provided it is administered together with Indomethacin (INDO) and cyclophosphamide (CY), in a vaccine immunotherapy regime. The present invention therefore comprises use of a natural cytokine mixture for use in immunotherapy of a cancer patient in a dosage regime as set in claim 1. Preferred features of the present invention are set out in the subsidiary claims.

Further background is given in the parent application, EP No 04780361.4 (granted as EP patent number 1653912), which is derived from PCT application US2004/025518, which was published as No WO2005/025494, the full contents of which documents are incorporated herein by way of reference.

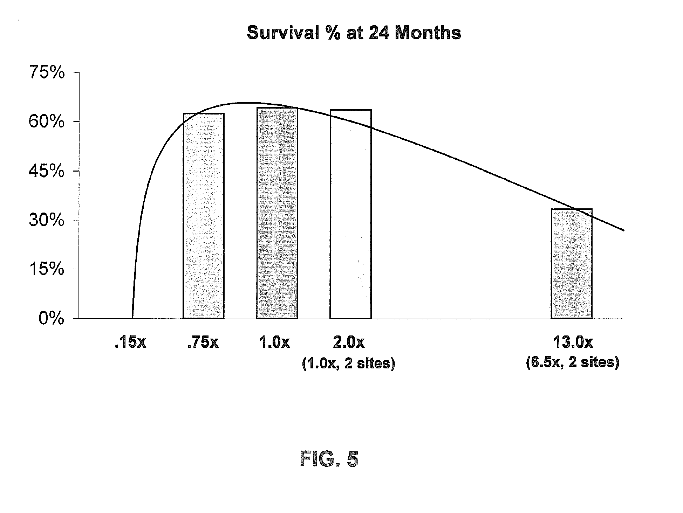

The abovementioned Figure 5 illustrates a series of doses of NCM on overall survival for 24 months. An optimal impact on survival is over the range 100 to 233 equivalents, as this amount has optimal impact on survival. No effect was observed at about 16 units and less effect at about 1320 units.

As previously described in the abovementioned WO02/34119 and WO03/035004, the NCM for use according to the invention is preferably for injection around lymphatics that drain into lymph nodes regional to a tumour.

The NCM is preferably co-delivered with CY and an NSAID. The NSAID of choice is indomethacin (INDO). However, ibuprofen, celecoxib, rofecoxib and other Coxll inhibitors can also be used as the NSAID. Side effects of NSAIDs should be aggressively treated with proton inhibitors and a prostaglandin E analog.

Zinc and multi-vitamins are useful agents to help restore T cell immunity, possibly including the addition of selenium. Preferable dosing of zinc is 50 to 75 mg. A standard multivitamin can be administered. The zinc can be an available gluconate.

The present invention will now be further illustrated with reference to the following worked example.

### EXAMPLE

### Dose and Frequency of the Natural Cytokine Mixture:

A ten day injection protocol was compared to twenty day injection protocol. Bilateral injections were compared to unilateral injections and a series of doses were compared. Significant activity on survival was observed from 74- 1310 units of IL-2 equivalence (as measured by ELISA, R & D Systems) with a peak at 100-233 units (Figure 1). Bilateral injections were effective and in one recurrent patient who had undergone an ipsilateral lymph node dissection, contralateral injection was used and a good response was obtained. This observation signifies that tumour antigen may also reside in contralateral nodes and thus bilateral injections are favoured. The twenty-day injection protocol was effective in terms of clinical response and survival yet the surgical specimen showed less lymphoid infiltration (17% area) than the ten-day unilateral protocol (34% area) or ten day bilateral protocol (33% area) (p < . 05). Less lymphoid infiltration with equivalent tumour reduction responses signifies that antibody- mediated immune responses are involved, since these responses are considered less effective than lymphocyte-mediated responses (i.e., cytotoxic T-cells) and the twenty-day injection protocol involves more labuor and expense, a ten-day bilateral injection protocol with 100 units of IL-2 equivalence or site is deemed optimal.

Figure 2 illustrates a series of doses of the NCM of the present invention on overall survival at twenty-four months. An optimal impact on survival at about 100-233 international units of IL-2 equivalence and no effect at about 16 units and less effect at about 1310 units.

### Role of the Nonsteroidal anti Inflammatory Drug (NSAIDs):

INDO is the most potent of NSAIDs acting on both cyclooxygenase I & II, but has greater gastrointestinal toxicity. Use of the two agents in place of INDO in a small series of patients gave lesser responses as measured by clinical and pathological criteria and by survival. In the case of Vioxx, all seven patients had clinical signs of gastritis following a week of therapy. In the cervical cancer patients, Ibuprofen was used as the NSAID and good responses were obtained.

Based upon these observations INDO is preferred, but Celebrex or Ibuprofen can be substituted if INDO is not tolerated. Vioxx is not recommended. Prilosec or other proton pump inhibitors with or without an oral prostaglandin analog is recommended as prophylaxis for gastritis, while histamine H2 blockers are not considered indicative.

### Role of the NSAID in Conjunction with CY:

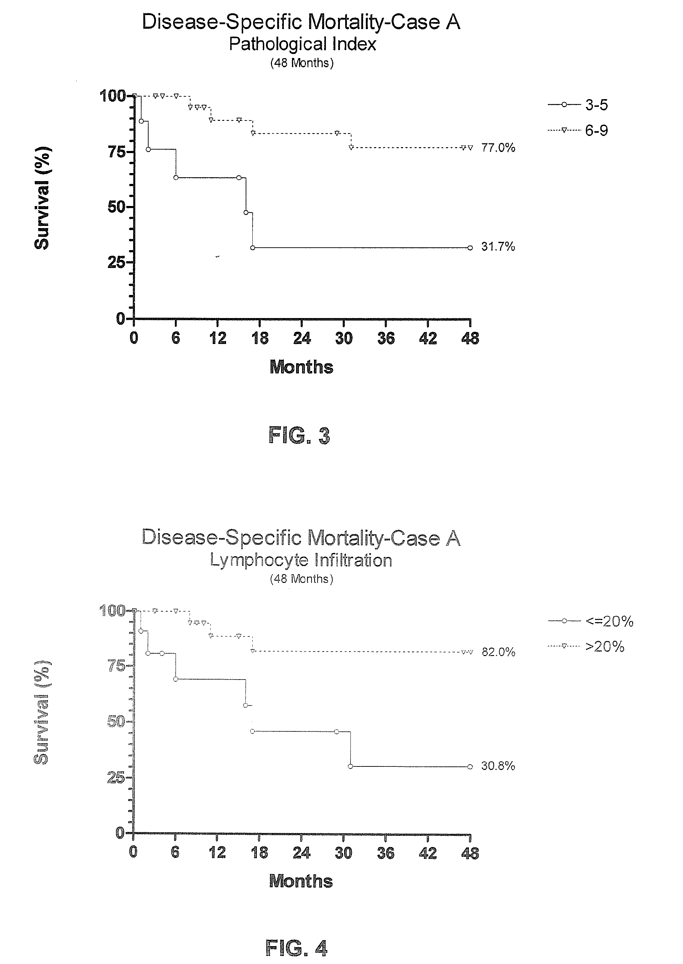

In four patients a dose of the NCM was given that was considered inactive (See, Figure 3, 15 units column) in conjunction with INDO and CY. No survivals were observed, yet two patients had minor response (< 50%, but > 25% tumour shrinkage) and all four showed moderate pathological changes in the tumour specimen with tumour reduction and fragmentation as well as lymphoid infiltration (See Table I, below). INDO can increase lymphoid infiltration and tumour reduction in some patients (See, Panje, 1981, and Hirsch, et al., 1983), but it has not been accepted clinically as a useful therapy in H & N SCC. Similarly, CY at this dose is not considered clinically active in H & N SCC. The activity of INDO and CY alone can be considered surprising in the magnitude and type of tumour response.

INDO and CY are therefore considered as a synergistic combination for employment with other forms of immunotherapy.

Recently low dose recombinant IL-2 was reported to delay recurrence of metastasis and increase mean survival time in patients with H & N SCC (See, DeStefani, et al., 2002, and Valente, et al, 1990). In the prior art research, no clinical responses were observed and lesser tumour changes (lymphoid infiltration without tumour regression) were observed. Nevertheless, rIL-2 can act with CY & INDO to further induce clinical responses and improve survival.

Other natural or recombinant cytokines corresponding to those present in the NCM singly or in combination are also potentially active. For example, cytokines such as IL-1, IFN-gamma, TNF alpha, IL-6, GM-CSF, G-CSF, IL-12, and combinations thereof can be used in natural or recombinant form.

**Table 1 CY & INDO (±NCM)**

| Patient No | Clinical response | %Tumour | %Solid | %Fragment | %Stoma | %Lymph | % Tumour Reduction |
|---|---|---|---|---|---|---|---|
| 17 | MR | 20 | 0 | 20 | 0 | 80 | 79 |
| 18 | MR | 60 | 15 | 45 | 0 | 40 | 33 |
| 19 | NR | 45 | 0 | 45 | 15 | 40 | 35 |
| 20 | NR | 70 | 15 | 42 | 15 | 15 | 5 |
| mean | | 49±11 | 11±7 | 36±6 | 8±4 | 44±13 | 38±16 |
| | | | | | | | |

| **Patient population** | **% tumour** | **% solid** | **% fragment** | | **% stroma** | **% lymph** | **% tumour reduction** |
|---|---|---|---|---|---|---|---|
| **On-protocol** | **49±11** | **22±4** | **26±4** | | **19±5** | **32±5** | **57** |
| **Untreated controls** | **80** | **80** | **0** | | **20** | **±** | **0** |

## Claims

1. Use of a natural cytokine mixture (NCM) including the cytokines IL-1, IL-2, IL-6, IL-8, IL-12, IFN-gamma, TNF-alpha, GM-CSF and G-CSF, for immunotherapy of a cancer patient in a dosage regime comprising
(i) administration of said NCM in an amount such that said NCM is inactive, and
(ii) administration of Indomethacin and cyclophosphamide.

2. Use according to claim 1, wherein no more than 15 units of said IL-2 are employed in said administration.

3. Use according to claim 1 or 2, wherein said administration precedes surgery, with or without radiotherapy.
